# EUROPÄISCHE PATENTSCHRIFT

(11) **EP 0 850 922 B1**
(45) Veröffentlichungstag und Bekanntmachung des Hinweises auf die Patenterteilung: **21.02.2001**
(21) Anmeldenummer: 97121628.8
(22) Anmeldetag: 09.12.1997
(51) Int. Cl.: C07C 319/08, C07C 321/04

(54) **Verfahren zur kontinuierlichen Herstellung von Methylmercaptan**
Process for the continuous preparation of methyl mercaptan
Procédé de préparation continue de méthylmercaptan

(30) Priorität: 27.12.1996 DE 19654515
(43) Veröffentlichungstag der Anmeldung: 01.07.1998
(73) Patentinhaber: Degussa-Hüls Aktiengesellschaft, 60287 Frankfurt am Main (DE)
(72) Erfinder: Hofen, Willi, 63517 Rodenbach (DE); Böck, Wolfgang, Dr., 63505 Langenselbold (DE); Rautenberg, Stephan, Dr., 63457 Hanau (DE); Sauer, Jörg, Dr., 63517 Rodenbach (DE); Arntz, Dietrich, Dr., Mobile, Al 36695 (US); Goedecke, Ralf, Dr., 63517 Rodenbach (DE); Taugner, Wolfgang, 63674 Altenstadt (DE); Sonnenschein, Raymund, Dr., 63526 Erlensee (DE)

(56) Entgegenhaltungen:
- DE-B- 1 134 368
- FR-A- 2 253 013
- FR-A- 2 477 538
- GB-A- 810 017

## Beschreibung

Die Erfindung betrifft ein Verfahren zur kontinuierlichen Herstellung von Methylmercaptan durch Umsetzen eines Eduktgasgemisches aus Methanol und Schwefelwasserstoff in der Dampfphase bei einer Reaktionstemperatur zwischen 300 und 500°C und unter einem Betriebsdruck von 5 bis 15 bar an einem Katalysatorbett auf Basis von Aluminiumoxid mit anschließender absorptiver und destillativer Abtrennung des Methylmercaptans vom Produktgasgemisch und Kreisführung von nicht verbrauchtem Methanol und Schwefelwasserstoff sowie Ausschleusung von Inertgasen und Abwasser und Ersetzen von verbrauchtem Methanol und Schwefelwasserstoff.

Methylmercaptan ist ein industriell wichtiges Zwischenprodukt für die Synthese von Methionin sowie für die Herstellung von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drucken zwischen 1 und 25 bar. Zur Erhöhung von Aktivität und Selektivität des Katalysators wird dieser üblicherweise mit Kaliumwolframat als Promotor belegt. Die Umsetzung von Schwefelwasserstoff und Methanol zu Methylmercaptan ist ein exothermer Prozeß, bei dem pro Kmol umgesetztes Methanol 28.500 KJ frei werden.

Das Produktgasgemisch enthält neben dem gebildeten Methylmercaptan und Wasser die nicht umgesetzten Ausgangsstoffe Methanol und Schwefelwasserstoff und als Nebenprodukte Dimethylsulfid und Dimethylether sowie in geringen Mengen auch Polysulfide. Darüber hinaus enthält das Produktgasgemisch die im Sinne der Reaktion inerten Gase Kohlendioxid, Kohlenmonoxid, Stickstoff und Wasserstoff.

Aus dem Produktgasgemisch wird das gebildete Methylmercaptan, wie in der DE 17 68 826 gezeigt, in mehreren Destillations- und Waschkolonnen bei Temperaturen zwischen 10 und 140°C abgetrennt. Dabei fallen als weitere Produktströme überschüssiger Schwefelwasserstoff, Methanol, die Inertgase und Abwasser an. Als Waschflüssigkeit wird bevorzugt Methanol verwendet.

Überschüssiger Schwefelwasserstoff wird als sogenanntes Kreisgas in den Druckreaktor zurückgeführt. Das Kreisgas enthält neben Schwefelwasserstoff noch Methanol, Methylmercaptan, Dimethylsulfid und weitere Stoffkomponenten aus dem Produktgasgemisch. Der Anteil dieser Komponenten am Kreisgas hängt von der Qualität des Trennprozesses ab. Ebenso wird das nicht verbrauchte Methanol wieder in das Eduktgasgemisch eingespeist. Auch das zurückgeführte Methanol enthält wie das Kreisgas noch weitere Stoffkomponenten. Verbrauchter Schwefelwasserstoff und Methanol werden durch Zuführen von frischen Medien ersetzt.

Der Gesamtprozeß der Methylmercaptan-Herstellung kann in zwei Abschnitte unterteilt werden. Der erste Abschnitt umfaßt die Aufbereitung des Eduktgasgemisches und seine Umsetzung zu Methylmercaptan. Der zweite Abschnitt beinhaltet die Auftrennung des Produktgasgemisches zur Gewinnung von Methylmercaptan und Rückführung von nicht verbrauchtem Methanol und Schwefelwasserstoff sowie Entsorgung von Abwasser und Abgasen. Die vorliegende Erfindung befaßt sich mit Verbesserungen im ersten Abschnitt des Herstellungsprozesses.

Die Art der Aufbereitung des Eduktgasgemisches, seine Aufheizung auf Reaktionstemperatur und die anschließende Kühlung des Produktgasgemisches zur Kondensation und Abtrennung des Methylrnercaptans haben einen wesentlichen Einfluß auf die Wirtschaftlichkeit des Gesamtprozesses. Es werden große elektrische Leistungen für den Betrieb der Verdichter sowie große Heiz- und Kühlleistungen benötigt.

Die DE 17 68 826 macht zu diesem ersten Abschnitt des Herstellungsprozesses nur geringfügige Angaben. Aus dem gezeigten Verfahrensschema ist zu entnehmen, daß das Kreisgas zusammen mit Schwefelwasserstoff-Frischgas in einem Wärmetauscher vom heißen Produktgasgemisch aufgeheizt wird. Dabei wird gleichzeitig das Produktgasgemisch gekühlt. Das für die Umsetzung benötigte Methanol wird nach Erhitzen des Schwefelwasserstoffes im Wärmetauscher kurz vor Eintritt in den Reaktor zur Bildung des Eduktgasgemisches dem Schwefelwasserstoff zugemischt. Hierfür wird das aus dem Produktgasgemisch abgetrennte Waschmethanol aus dem Waschmittelkreisstrom entnommen. Die aus dem Kreisstrom entnommene Menge wird durch frisches Methanol ersetzt.

Gemäß der FR 2 477 538 wird zur Herstellung von Methylmercaptan frisches Schwefelwasserstoffgas in einem Verdichter auf 11 bar verdichtet. Danach werden aus dem Prozeß zurückgeführtes Kreisgas, welches Schwefelwasserstoff, Dimethylsulfid, Methanol und geringe Mengen Methylmercaptan enthält, dem verdichteten Schwefelwasserstoff zur Bildung des Eduktgasgemisches zugemischt. Durch einen Vorwärmofen wird die Temperatur des Eduktgasgemisches auf 510°C angehoben. Vor Eintritt in den ersten von bis zu 10 hintereinandergeschalteten Reaktoren wird dem Eduktgasgemisch der Waschmittelkreisstrom, welcher Methanol und Dimethylsulfid enthält, zugemischt. Dadurch sinkt die Reaktoreingangstemperatur auf 450°C ab. Vor dem zweiten und den folgenden Reaktoren wird weiteres Methanol in den Gasstrom teilweise als Flüssigkeit und teilweise als Gas eingespritzt. Durch die benötigte Verfampfungswärme des Methanols kann dabei die gesamte oder ein Teil der Wärmemenge absorbiert werden, die bei der Reaktion frei wird.

Die DE-PS 11 34 368 beschreibt die Verwendung eines Rohrbündelreaktors für die Herstellung von Methylmercaptan. Der Rohrbündelreaktor besteht aus einem zylindrischen Behälter, in dem die Katalysatorröhren parallel zueinander angeordnet sind. Die Katalysatorröhren sind unten und oben mit Rohrabdeckplatten wie bei Rohrbündelwärmetauschern verschweißt. Die Zwischenräume zwischen den Röhren sind mit wärmeleitender Flüssigkeit gefüllt. Jede Katalysatorröhre ist an ihrem unteren Ende zum Beispiel mit einem Sieb versehen, welches den teilchenförmigen Katalysator trägt. Das Eduktgasgemisch durchströmt den Reaktor von unten nach oben.

Der Katalysator besteht aus aktiviertem Aluminiumoxid in Form von Kügelchen mit der Siebmaschengröße 8 bis 14. Im unteren Abschnitt der Katalysatorröhren wird der Katalysator vorzugsweise mit Teilchen aus inertem Stoff, wie Kieselsäure oder geschmolzenem Aluminiumoxid, verdünnt, wobei der inerte Stoff etwa 75 % der Teilchen im unteren Drittel der Röhren ausmacht. Von dieser Höhe ab nimmt die Menge an inertem Stoff zum oberen Abschnitt der Röhren hin ab, so daß im oberen Teil der Röhren reiner Katalysator vorliegt. Die allmählich verringerte Verdünnung des Katalysators in Strömungsrichtung ergibt eine gleichmäßigere Wärmeentwicklung, wodurch die Temperaturregelung erleichtert wird.

In der DE-PS 11 34 368 wird als wärmeleitende Flüssigkeit ein eutektisches Gemisch aus Phenylether und Diphenyl verwendet. Diese Kühlflüssigkeit verdampft infolge der Reaktionswärme und wird in einem Kühlmittelbehälter wieder kondensiert und in den Reaktor zurückgeführt. Gemäß der DE-PS 11 34 368 wird das Eduktgasgemisch im Wärmetausch mit dem heißen Produktgasgemisch und den heißen Kühlmitteldämpfen aufgeheizt.

Aufgabe der vorliegenden Erfindung ist es, ein Verfahren zur Herstellung von Methylmercaptan anzugeben, welches durch eine verbesserte Aufbereitung des Eduktgasgemisches und eine bessere Nutzung der Reaktionswärme die Wirtschaftlichkeit des Gesamtverfahrens bezüglich der Investitionskosten als auch der laufenden Energiekosten erhöht.

Die Erfindung wird durch ein gattungsgemäßes Verfahren gelöst, welches dadurch gekennzeichnet ist,
daß das Eduktgemisch durch folgende Verfahrensschritte gewonnen wird:
a) Verdichten von Schwefelwasserstoff-Frischgas auf einen Zwischendruck, der etwa dem halben Betriebsdruck entspricht, unter Zusatz von flüssigem Methanol,
b) Zumischen des im Kreis geführten Schwefelwasserstoffgases zum Frischgas und Verdichten des Gemisches auf den Betriebsdruck,
c) Zuführen von weiterem Methanol in Form von Methanoldampf zu dem verdichteten Gasgemisch zur Bildung des Eduktgasgemisches mit einem Molverhältnis von Schwefelwasserstoff zu Methanol von 1,1 bis 3,
d) Erwärmen des Eduktgasgemisches auf eine Vortemperatur im Bereich von 150 bis 200°C,
e) weiteres Erwärmen des Eduktgasgemisches auf die Reaktionstemperatur im Wärmetausch mit der am Katalysatorbett frei werdenden Reaktionswärme.

Erfindungsgemäß wird das Schwefelwasserstoff-Frischgas in einer ersten Verdichtungsstufe von Normaldruck zunächst auf einen Zwischendruck verdichtet. Dabei wird dem Schwefelwasserstoffgas flüssiges Methanol vor oder während des Verdichtungsvorgangs durch Einsprühen oder Einspritzen zugesetzt. Durch die bei der Verdichtung frei werdende Verdichtungswärme verdampft das Methanol zum Teil. Die Temperaturerhöhung des verdichteten Gases kann dadurch auf einen Wert unter 100°C begrenzt werden. Nach der Verdichtung ist der Gasstrom bei der hinter der ersten Verdichtungsstufe vorliegenden Temperatur mit Methanol abgesättigt.

Das aus dem Produktgasgemisch abgetrennte und im Kreis geführte Schwefelwasserstoffgas, im folgenden als Kreisgas bezeichnet, wird zusammen mit dem vorverdichteten Frischgas in einer zweiten Verdichtungsstufe vom Zwischendruck auf den Betriebsdruck verdichtet. Aufgrund der Temperaturbegrenzung in der ersten Verdichtungsstufe steigt die Gastemperatur nach der zweiten Verdichtungsstufe nur auf maximal 140°C.

Durch das Einspritzen von Methanol in die erste Verdichtungsstufe wird ein Teil der Verdichtungsenergie zur Verdampfung von Methanol unter gleichzeitiger Temperaturbegrenzung in der ersten Verdichtungsstufe ausgenutzt. Hierdurch werden Investitionskosten für einen sonst notwendigen Zwischenkühler sowie laufende Kosten für Kühlwasser vermieden. Weitere Einsparungen ergeben sich dadurch, daß ein Teil des für die Umsetzung benötigten Methanols durch die frei werdende Verdichtungswärme verdampft wird.

Nach der zweiten Verdichtungsstufe wird dem Eduktgas weiteres Methanol in Form von Methanoldampf zur Einstellung eines Molverhältnisses von Schwefelwasserstoff zu Methanol von 1,1 bis 3 zugeführt.

Ein hohes Molverhältnis begünstigt die katalytische Umsetzung von Schwefelwasserstoff und Methanol zu Methylmercaptan. Ein großer Überschuß von Schwefelwasserstoff bedeutet allerdings auch, daß große Mengen von Schwefelwasserstoff im Kreis geführt werden müssen. Zur Begrenzung des hierfür benötigten Energieaufwandes sollte daher das Molverhältnis den Wert 3 nicht übersteigen. Bei Werten von unter 1,1 sind dagegen die Umsätze zu Methylmercaptan auch bei Verwendung hoch aktiver und selektiver Katalysatoren unbefriedigend. Bevorzugt wird daher ein Molverhältnis von 1,5 bis 2,0 eingestellt.

Die in der ersten Verdichtungsstufe verdampfbare Menge Methanol hängt vom gewählten Zwischendruck und dem gewählten Molverhältnis ab. Je größer das Molverhältnis ist, um so mehr Schwefelwasserstoff muß relativ zum Methanol verdichtet werden und um so mehr Methanol kann verdampft werden. Es wird ein Zwischendruck gewählt, der etwa dem halben Betriebsdruck entspricht. In diesem Fall können zum Beispiel bei einem Molverhältnis von 1,8 und einem Zwischendruck von 6 bar in der ersten Verdichtungsstufe etwa 25 % des insgesamt für die Umsetzung benötigten Methanols verdampft werden.

Die Temperatur des so erhaltenen Eduktgasgemisches wird durch Zufuhr externer Wärme mittels eines Gaserhitzers auf eine Vortemperatur im Bereich zwischen 150 und 200°C angehoben, bevor es im Wärmetausch mit der am Katalysatorbett frei werdenden Reaktionswärme auf die eigentliche Reaktionstemperatur erwärmt und über das Katalysatorbett zur Umsetzung von Schwefelwasserstoff und Methanol zu Methylmercaptan geleitet wird.

In einer bevorzugten Ausführungsform der Erfindung wird die katalytische Umsetzung in einem Rohrbündelreaktor vorgenommen, dessen Rohre in Strömungsrichtung zuerst mit einer Inertschüttung und nachfolgend mit der Katalysatorschüttung gefüllt sind, wobei die an der Katalysatorschüttung frei werdende Reaktionswärme durch ein zwischen den Rohren umlaufendes Wärmeträgermedium an die stromaufwärts liegende Inertschüttung zur Aufheizung des Eduktgasgemisches auf Reaktionstemperatur übertragen wird. Hierzu wird das Wärmeträgermedium im Gegenstrom zur Strömungsrichtung des Eduktgasgemisches durch den Reaktorbehälter gepumpt und transportiert dadurch die an der Katalysatorschüttung frei werdende Reaktionswärme zur Inertschüttung. Als Wärmeträgermedium eignet sich zum Beispiel eine Salzschmelze. Die Längen von Inertschüttung und Katalysatorschüttung sowie die Vortemperatur (Reaktoreingangstemperatur) können in einfacher Weise so aufeinander abgestimmt werden, daß das Eduktgasgemisch nach Durchlaufen der Inertschüttung auf die Reaktionstemperatur von 300 bis 500°C aufgeheizt ist.

Diese Betriebsweise des Reaktors bietet vielfältige Vorteile. So muß der externe Gaserhitzer das Eduktgasgemisch nur auf maximal 200°C erwärmen. Er kann entsprechend einfach ausgelegt sein. Im Gegensatz dazu muß in der FR 2 477 538 das Eduktgasgemisch vor Eintritt in den Reaktor auf 510°C aufgeheizt werden. Das erfordert einen teuren Gaserhitzer mit großer Heizleistung. Er muß aus korrosionsfesten Materialien gefertigt sein, denn bekanntermaßen wirkt Schwefelwasserstoff bei Temperaturen über 500°C stark korrosiv.

Durch den internen Wärmetausch im Reaktor kann auf Maßnahmen zur Abführung der Reaktionswärme verzichtet werden. Die zwecks Temperaturregelung des Reaktors notwendigen Heiz- und Kühlvorrichtungen für das umlaufende Wärmeträgermedium können entsprechend klein ausgelegt werden. Dagegen wird gemäß der DE-PS 11 34 368 die gesamte Reaktionswärme mit Hilfe des Kühlmittels nach außen abgeführt und in einem separaten Wärmetauscher an das Eduktgasgemisch abgegeben.

In einer weiteren bevorzugten Ausführungsform der Erfindung wird die zur Methanolverdampfung benötigte Energie mit Hilfe eines Wärmetauschers dem Produktgasgemisch nach Verlassen des Druckreaktors entzogen. Dabei kühlt sich das Produktgasgemisch auf 100 bis 150°C ab. Die in dem Produktgasgemisch enthaltene Enthalpie reicht bei Ausnutzung bis 150°C aus, um die gesamte im Prozeß benötigte Methanolmenge zum Beispiel bei einer Temperatur von 137°C und 10 bar zu verdampfen. Durch die Kopplung der Methanolverdampfung an die Reaktionswärme stellt sich das für die Umsetzung benötigte Molverhältnis von Schwefelwasserstoff zu Methanol im wesentlichen automatisch ein.

Die in zwei Verdichtungsstufen aufgeteilte Verdichtung der Eduktgase auf Betriebsdruck wird bevorzugt mit einem zweistufigen Verdichter vorgenommen, wobei das Gasgemisch in der ersten Stufe auf den Zwischendruck und in der zweiten Stufe auf den Betriebsdruck verdichtet wird. Besonders geeignet hierzu sind zweistufige Schraubenverdichter. Diese Verdichter sind sehr kompakt und robust. Das Methanol kann direkt in die erste Verdichtungsstufe eingespritzt werden. Es hat sich dabei als vorteilhaft erwiesen, Methanol im Überschuß einzuspritzen, das heißt mehr Methanol zuzuführen als durch die Verdichtungswärme verdampft werden kann. Das überschüssige, nicht verdampfte Methanol wird am Ausgang der ersten Verdichterstufe ausgeschleust und an den Eingang zurückgeführt. Durch diesen Anteil umlaufenden, flüssigen Methanols wird die erste Verdichterstufe gespült und von gröberen Schwefelablagerungen befreit. Die mit dem Methanol ausgeschwemmten Partikel werden in einem Filter abgeschieden. Das Verhältnis des verdampften zum nicht verdampften Methanol kann in weiten Grenzen variiert werden. Bewährt hat sich ein Gewichtsverhältnis von 2 : 1.

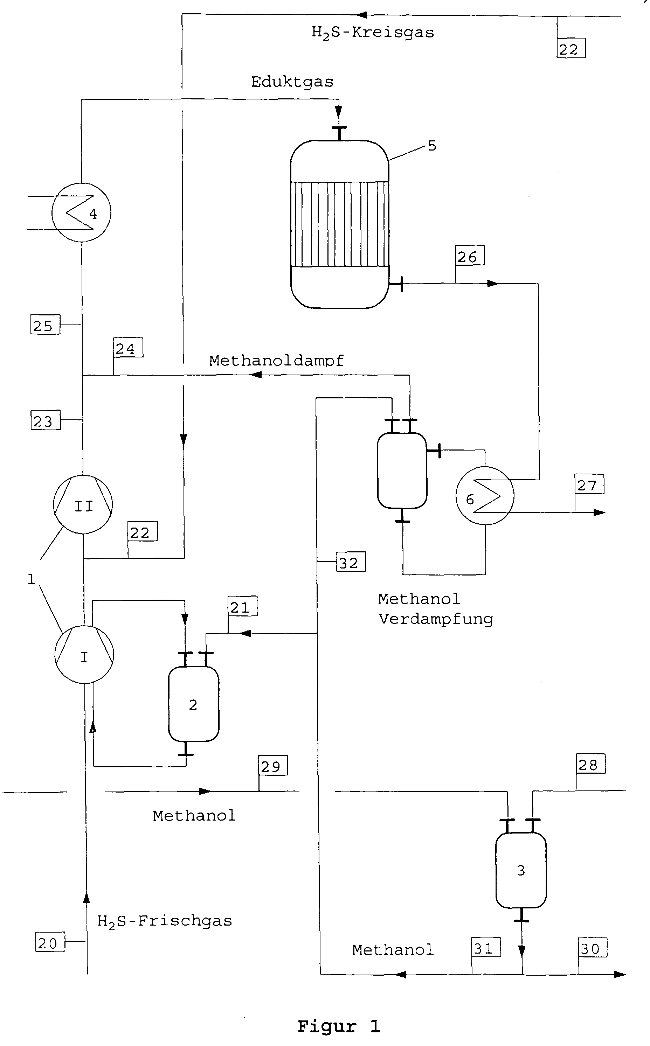

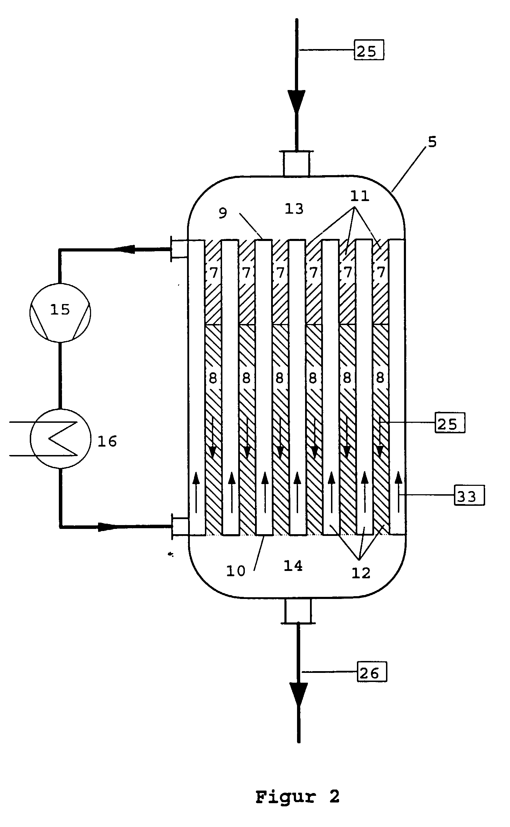

Figur 1 und Figur 2 dienen zur weiteren Erläuterung der Erfindung. Es zeigen:
- Figur 1:: Verfahrensschema für den ersten Abschnitt des Herstellungsprozesses von Methylmercaptan.
- Figur 2:: Detaildarstellung eines Rohrbündelreaktors mit Inert- und Katalysatorschüttung und Umlauf des Wärmeträgermediums.

Figur 1 zeigt ein Verfahrensschema für den ersten Abschnitt der Methylmercaptan-Herstellung, welcher die Eduktgas-Aufbereitung, die Umsetzung im Reaktor und die Kühlung des Produktgasgemisches umfaßt.

Die Umsetzung im Rohrbündelreaktor 5 erfolgt an einem Katalysator aus Aluminiumoxidgranulat, welcher mit 25 Gew.-% Cäsiumwolframat belegt ist. Die Korngröße des Granulats beträgt etwa 3 mm. Dieser Katalysator ist detailliert in der nicht vorveröffentlichten deutschen Patentanmeldung DE 196 39 584, Beispiel 2, beschrieben. Er ist in der Lage, ein Eduktgasgemisch mit einem Molverhältnis von Schwefelwasserstoff zu Methanol von 1,5 : 1 bis 2,0 : 1 bei einem Betriebsdruck von 10 bar, einer Reaktionstemperatur zwischen 340 und 370°C und bei einer Belastung mit einer Raumgeschwindigkeit GHSV von 800 bis 1200 h⁻¹ mit einem Methanolumsatz und einer Selektivität von jeweils mehr als 90 % zu Methylmercaptan umzusetzen.

Zur Bereitstellung des Eduktgasgemisches wird das Schwefelwasserstoff-Frischgas 20 in der ersten Stufe I eines zweistufigen Schraubenverdichters 1 unter gleichzeitigem Einspritzen von flüssigem Methanol auf den Zwischendruck von 6 bar verdichtet. Durch die Verdichtungswärme wird ein Teil des Methanols verdampft und begrenzt dadurch die Temperatur nach der ersten Verdichtungsstufe auf einen Wert von etwa 65°C. Das durch die Verdichtungswärme nicht verdampfte Methanol wird über den Pufferbehälter 2 im Kreis geführt und durch geeignete Filter von Partikeln, welche aus dem Verdichter ausgeschwemmt wurden, befreit. Der verdampfte Mengenstrom Methanol entspricht bei einem Molverhältnis von 1,5 : 1 bis 2,0 : 1 etwa 20 bis 30 % des für die Umsetzung benötigten Methanols und wird durch den Mengenstrom 21 aus einem weiteren Pufferbehälter 3 ersetzt.

Dem auf den Zwischendruck verdichteten Frischgas wird ein Strom 22 von Schwefelwasserstoff-Kreisgas zugemischt.

Anschließend wird dieses Gasgemisch in der zweiten Verdichterstuffe II des Schraubenverdichters auf 11 bar verdichtet. Dieser Enddruck des Schraubenverdichters liegt zum Ausgleich von Leitungsverlusten etwas über dem eigentlichen Betriebsdruck im Reaktor. Infolge der Verdichtungswärme steigt die Temperatur des Gasgemisches auf etwa 100 bis 110°C an.

Zur Einstellung des gewünschten Molverhältnisses von Schwefelwasserstoff zu Methanol wird dem verdichteten Gasstrom 23 Methanoldampf 24 zugeführt. Er weist eine Temperatur von etwa 135 bis 150°C bei einem Druck von 11 bar auf.

Das so erhaltene Eduktgasgemisch 25 wird im Gaserhitzer 4 auf die Reaktoreingangstemperatur (Vortemperatur) von 150 bis 200°C erwärmt. Mit dieser Temperatur gelangt das Eduktgasgemisch in den Reaktor 5 und wird dort im Wärmetausch mit der am Katalysatorbett frei werdenden Reaktionswärme auf die Reaktionstemperatur erwärmt.

Das Produktgasgemisch 26 verläßt den Reaktor mit der Reaktionstemperatur. Sein Wärmeinhalt wird im Wärmetauscher 6 zur Methanolverdampfung ausgenutzt. Dabei kühlt sich das Produktgasgemisch auf etwa 150°C ab und wird als Mengenstrom 27 dem zweiten Verfahrensabschnitt zugeführt. Die Auftrennung des Produktgasgemisches in seine Komponenten wird im zweiten Verfahrensabschnitt der Methylmercaptan-Herstellung vorgenommen. Die Auftrennung kann nach verschiedenen, bekannten Verfahren erfolgen. Eine besonders vorteilhafte Auftrennung des Produktgasgemisches wird in der parallelen Patentanmeldung EP 0850923 A1 beschrieben.

Wichtig ist, daß das im zweiten Verfahrensabschnitt abgetrennte Schwefelwasserstoffgas als Kreisgasstrom 22 zurückgeführt wird. Gleiches gilt für das vom Produktgasgemisch abgetrennte, bei der Umsetzung im Reaktor nicht vollständig verbrauchte Methanol, sowie für das im zweiten Verfahrensabschnitt gegebenenfalls verwendete Waschmethanol. Beide Methanolanteile werden als Mengenstrom 28 in den Pufferbehälter 3 zurückgeführt.

Im Herstellungsprozeß verbrauchtes Methanol wird durch frisches Methanol ersetzt, welches als Mengenstrom 29 dem Pufferbehälter 3 zugeführt wird.

Aus dem Pufferbehälter 3 wird ein Mengenstrom 30 für die Methanolwäsche im zweiten Verfahrensabschnitt und ein Methanolstrom 31 für die katalytische Umsetzung entnommen. Der Methanolstrom 31 wird in die beiden Teilströme 21 und 32 aufgeteilt, wobei Strom 21 in der ersten Verdichterstufe verdampft wird und Strom 32 im Wärmetausch mit dem heißen Produktgasgemisch in die Dampfphase überführt wird.

Figur 2 zeigt die bevorzugte Ausführungsform des Reaktors gemäß Anspruch 2. In dem Reaktor 5 sind zwischen zwei Rohrabdeckplatten 9 und 10 die Katalysatorrohre 11 parallel zueinander eingeschweißt. Das Eduktgasgemisch 25 tritt über den Verteilerraum 13 in die Katalysatorrohre ein. Die Katalysatorrohre sind in Strömungsrichtung des Eduktgases zunächst mit einer Inertschüttung 7 aus Keramik-Raschigringen und anschließend mit der Katalysatorschüttung 8 gefüllt. Nach Verlassen der Katalysatorrohre wird das umgesetzte Gasgemisch über den Sammelraum 14 als Produktgasstrom 26 der weiteren Verarbeitung zugeführt.

Die Zwischenräume 12 zwischen den Katalysatorrohre sind mit einer Salzschmelze aus Kaliumnitrat und Kaliumnitrit (Schmelztemperatur etwa 150°C) als Wärmeträgermedium 33 gefüllt. Das Wärmeträgermedium wird im Gegenstrom zu den Eduktgasen durch den Reaktor geführt. Hierzu wird das Wärmeträgermedium unterhalb der Abdeckplatte 9 aus dem Reaktorbehälter ausgekoppelt und über einen äußeren Kreislauf oberhalb der Abdeckplatte 10 wieder in die Zwischenräume 12 eingespeist. Mit 15 ist die für den Wärmeträgerkreislauf notwendige Umwälzpumpe bezeichnet.

Durch die Umwälzung des Wärmeträgermediums wird die an der Katalysatorschüttung 8 frei werdende Reaktionswärme im Bereich der Inertschüttung 7 an das Eduktgasgemisch übertragen. Zur Temperaturregelung des Reaktors ist ein Wärmetauscher 16 zur Aufheizung und Kühlung des Wärmeträgermediums vorgesehen.

Figur 2 zeigt die bevorzugte Orientierung des Rohrbündelreaktors. Die Rohre sind vertikal angeordnet und das Eduktgasgemisch durchströmt den Reaktor von oben nach unten. Die Katalysatorrohre sind an ihrer unteren Öffnung mit einem geeigneten Sieb als Träger für die Katalysatorschüttung versehen. Der Reaktor kann auch eine beliebige andere Orientierung aufweisen.

Die Reaktoreingangstemperatur des Eduktgasgemisches beträgt im stationären Betrieb etwa 170°C. Zur Erwärmung des Eduktgases in der Inertschüttung 7 auf die Reaktionstemperatur von 360°C beträgt die Länge der Inertschüttung etwa 15 % der Gesamtlänge der Katalysatorrohre.

Der aus dem Reaktor austretende Produktgasstrom 26 hat bei einem Molverhältnis von 1,8 (Schwefelwasserstoff/Methanol) folgende typische Zusammensetzung:

| | |
|---|---|
| Methylmercaptan | 39 Gew.-% |
| Dimethylsulfid | 1,6 Gew.-% |
| Dimethylether | 2,7 Gew.-% |
| Inertgase (H₂, CO, CO₂, N₂) | 2,5 Gew.-% |
| Wasser: | 15 Gew.-% |
| Schwefelwasserstoff | 34 Gew.-% |
| Methanol | 5 Gew.-% |

Das in Figur 1 dargestellte Verfahrensschema enthält die für die Durchführung des erfindungsgemäßen Verfahrens notwendigen Komponenten. Nicht dargestellt sind zusätzliche Komponenten, die als Anfahrhilfen für die Inbetriebnahme des Verfahrens benötigt werden. Hierzu gehört ein dampfbeheizter Methanolverdampfer, der, solange die katalytische Umsetzung noch nicht in Gang gekommen ist, an Stelle des Produktgases die notwendige Menge Methanol verdampft. Während dieser Anfahrphase wird auch der Wärmetauscher 16 zur Aufheizung des Reaktors verwendet. Gegebenenfalls kann hier eine zusätzliche, elektrische Beheizung vorgesehen werden.

Das erfindungsgemäße Verfahren zeichnet sich durch eine optimale Nutzung der im Prozeß frei werdenden Energieströme aus. Dabei werden diese Energieströme überwiegend direkt in der jeweiligen Verfahrensstufe genutzt, in der sie anfallen. Dadurch werden Investitionskosten für externe Wärmetauscher vermieden. So wird zum Beispiel ein Teil des benötigten Methanols unter Ausnutzung der Verdichtungswärme bei der Verdichtung des Schwefelwasserstoff-Frischgases verdampft. Hierzu wird das Methanol direkt in den Verdichter eingespritzt und macht dadurch eine zusätzliche Kühlung für den Verdichter überflüssig.

Das Eduktgasgemisch wird direkt im Reaktor selber mit Hilfe der am Katalysatorbett frei werdenden Reaktionswärme auf die Reaktionstemperatur aufgeheizt. Ein externer Gaserhitzer wird nur benötigt, um das Eduktgasgemisch auf eine relativ geringe Reaktoreingangstemperatur zu erwärmen und kann daher dementsprechend einfach ausgelegt sein.

## Patentansprüche

1. Verfahren zur kontinuierlichen Herstellung von Methylmercaptan durch Umsetzen eines Eduktgasgemisches aus Methanol und Schwefelwasserstoff in der Dampfphase bei einer Reaktionstemperatur zwischen 300 und 500°C und unter einem Betriebsdruck von 5 bis 15 bar an einem Katalysatorbett auf Basis von Aluminiumoxid mit anschließender absorptiver und destillativer Abtrennung des Methylmercaptans vom Produktgasgemisch und Kreisführung von nicht verbrauchtem Methanol und Schwefelwasserstoff sowie Ausschleusung von Inertgasen und Abwasser und Ersetzen von verbrauchtem Methanol und Schwefelwasserstoff,
**dadurch gekennzeichnet,**
daß das Eduktgasgemisch durch folgende Verfahrensschritte gewonnen wird:
a) Verdichten von Schwefelwasserstoff-Frischgas auf einen Zwischendruck, der etwa dem halben Betriebsdruck entspricht, unter Zusatz von flüssigem Methanol,
b) Zumischen des im Kreis geführten Schwefelwasserstoffgases zum Frischgas und Verdichten des Gemisches auf den Betriebsdruck,
c) Zuführen von weiterem Methanol in Form von Methanoldampf zu dem verdichteten Gasgemisch zur Bildung des Eduktgasgemisches mit einem Molverhältnis von Schwefelwasserstoff zu Methanol von 1,1 bis 3,
d) Erwärmen des Eduktgasgemisches auf eine Vortemperatur im Bereich von 150 bis 200°C,
e) weiteres Erwärmen des Eduktgasgemisches auf die Reaktionstemperatur im Wärmetausch mit der am Katalysatorbett frei werdenden Reaktionswärme und
f) Umsetzen von Methanol und Schwefelwasserstoff zu Methylmercaptan am Katalysatorbett.

2. Verfahren nach Anspruch 1,
**dadurch gekennzeichnet,**
daß die Umsetzung in einem Rohrbündelreaktor durchgeführt wird, dessen Rohre in Strömungsrichtung zuerst mit einer Inertschüttung und nachfolgend mit der Katalysatorschüttung gefüllt sind, wobei die an der Katalysatorschüttung frei werdende Reaktionswärme durch ein zwischen den Rohren umlaufendes Wärmeträgermedium an die stromaufwärts liegende Inertschüttung zur Aufheizung des Eduktgasgemisches auf Reaktionstemperatur übertragen wird.

3. Verfahren nach Anspruch 1 oder 2,
**dadurch gekennzeichnet,**
daß die für die Verdampfung des weiteren Methanols benötigte Wärmemenge dem Produktgasgemisch unter gleichzeitiger Abkühlung des Produktgasgemisches auf 100 bis 150°C entzogen wird.

4. Verfahren nach einem der vorstehenden Ansprüche,
**dadurch gekennzeichnet,**
daß das Eduktgasgemisch mit einem zweistufigen Verdichter verdichtet wird, wobei das Gasgemisch in der ersten Stufe auf den Zwischendruck und in der zweiten Stufe auf den Betriebsdruck verdichtet wird.

5. Verfahren nach Ansprch 4,
**dadurch gekennzeichnet,**
daß als Verdichter ein zweistufiger Schraubenverdichter verwendet und das flüssige Methanol direkt in die erste Verdichterstufe eingespritzt wird.

6. Verfahren nach Anspruch 5,
**dadurch gekennzeichnet,**
daß das flüssige Methanol im Überschuß eingespritzt und der nicht verdampfte Anteil im Kreis geführt wird.

## Claims

1. Process for the continuous production of methyl mercaptan by reacting an educt gas mixture of methanol and hydrogen sulfide in the vapour phase at a reaction temperature of between 300 and 500°C and under an operating pressure of 5 to 15 bar on a catalyst bed based on aluminium oxide with subsequent separation of the methyl mercaptan from the product gas mixture by absorption and distillation and recirculation of unconsumed methanol and hydrogen sulfide and discharge of inert gases and waste water and replacement of the methanol and hydrogen sulfide which has been consumed,
characterised in that
the educt gas mixture is obtained by the following process steps:
a) compression of fresh hydrogen sulfide gas to an intermediate pressure, corresponding to approx. half the operating pressure, with addition of liquid methanol,
b) mixing of the recirculated hydrogen sulfide gas with the fresh gas and compression of the mixture to the operating pressure,
c) introduction of further methanol in the form of methanol vapour into the compressed gas mixture to form the educt gas mixture with an molar ratio of hydrogen sulfide to methanol of 1.1 to 3,
d) heating of the educt gas mixture to an initial temperature in the range from 150 to 200°C,
e) further heating of the educt gas mixture to the reaction temperature by heat exchange with the heat of reaction liberated on the catalyst bed and
f) reaction of methanol and hydrogen sulfide on the catalyst bed to yield methyl mercaptan.

2. Process according to claim 1,
characterised in that
the reaction is performed in a multi-tube flow reactor, the tubes of which are filled in the direction of flow first with an inert packing and then with the catalyst packing, wherein the heat of reaction liberated on the catalyst packing is transferred by a heat transfer medium circulating between the tubes to the upstream inert packing in order to heat the educt gas mixture to the reaction temperature.

3. Process according to claim 1 or 2,
characterised in that
the quantity of heat required to vaporise the further methanol is drawn from the product gas mixture, with the product gas mixture simultaneously being cooled to 100 to 150°C.

4. Process according to one of the preceding claims,
characterised in that
the educt gas mixture is compressed with a two-stage compressor, wherein the gas mixture is compressed to the intermediate pressure in the first stage and to the operating pressure in the second stage.

5. Process according to claim 4,
characterised in that
a two-stage screw compressor is used as the compressor and the liquid methanol is injected directly into the first compressor stage.

6. Process according to claim 5,
characterised in that
the liquid methanol is injected in excess and the unvaporised fraction is recirculated.

## Revendications

1. Procédé de production en continu de méthylmercaptan par conversion d'un mélange gazeux d'éduit à base de méthanol et d'acide sulfhydrique en phase vapeur, à une température de réaction comprise entre 300 et 500°C et à une pression de fonctionnement de 5 à 15 bars sur un lit de catalyseur à base d'oxyde d'aluminium avec séparation consécutive par absorption et par distillation du méthylmercaptan, du mélange gazeux produit et recyclage du méthanol non consommé et de l'acide sulfhydrique ainsi qu'évacuation des gaz inertes et des eaux usées et remplacement du méthanol consommé et de l'acide sulfhydrique,
caractérisé en ce que
le mélange gazeux d'éduit est produit grâce aux étapes de procédé suivantes :
a) compression du gaz frais d'acide sulfhydrique à une pression intermédiaire qui correspond à environ la moitié de la pression de fonctionnement, tout en ajoutant du méthanol liquide,
b) mélange de l'acide sulfhydrique gazeux recyclé au gaz frais et compression du mélange à une pression de fonctionnement,
c) amenée de méthanol en supplément sous forme de vapeur de méthanol au mélange gazeux comprimé en vue de la formation du mélange gazeux d'éduit avec un rapport molaire de l'acide sulfhydrique au méthanol de 1,1 à 3,
d) chauffage du mélange gazeux d'éduit à une température préalable dans la plage de 150 à 200°C,
e) chauffage supplémentaire du mélange gazeux d'éduit à la température de réaction par échange de chaleur avec la chaleur de réaction dégagée sur le lit de catalyseur, et
f) conversion du méthanol et de l'acide sulfhydrique en méthylmercaptan sur le lit de catalyseur.

2. Procédé selon la revendication 1,
caractérisé en ce qu'
on effectue la conversion dans un réacteur à faisceau de tubes, dont les tubes sont chargés dans la direction du courant en premier lieu avec du ballast et ensuite avec le catalyseur en vrac, pour lequel la chaleur de réaction dégagée sur le catalyseur en vrac est transférée à l'aide d'un milieu caloporteur circulant entre les tubes, sur le ballast qui se trouve en amont, en vue du chauffage du mélange gazeux d'éduit à la température de réaction.

3. Procédé selon la revendication 1 ou 2,
caractérisé en ce que
la quantité de chaleur requise pour la vaporisation du méthanol supplémentaire est retirée du mélange gazeux produit, tout en refroidissant simultanément le mélange gazeux produit de 100 à 150°C.

4. Procédé selon l'une des revendications précédentes,
caractérisé en ce que
le mélange gazeux d'éduit est comprimé avec un compresseur à deux étages, dans lequel le mélange gazeux est comprimé dans le premier étage à la pression intermédiaire et dans le deuxième étage à la pression de fonctionnement.

5. Procédé selon la revendication 4,
caractérisé en ce que
comme compresseur on utilise un compresseur à vis à deux étages et on injecte le méthanol liquide directement dans le premier étage de compresseur.

6. Procédé selon la revendication 5,
caractérisé en ce que
le méthanol liquide est injecté en excès, et la fraction qui n'est pas vaporisée est remise dans le circuit.
